# EUROPEAN PATENT APPLICATION

(11) **EP 4 521 365 A1**
(43) Date of publication of application: **12.03.2025**
(21) Application number: 23195792.9
(22) Date of filing: 06.09.2023
(51) Int. Cl.: G06V 20/70, G06F 18/21, G06V 10/778, G06V 20/40, G06T 7/00

(54) **DETERMINING TYPES OF MICROSURGICAL INTERVENTIONS**

(71) Applicant: Carl Zeiss Meditec AG, 07745 Jena (DE)
(72) Inventor: FREYTAG, Alexander, 73447 Oberkochen (DE); GHAZAEI, Ghazal, 73447 Oberkochen (DE); ALPEEV, Dmitry, 73447 Oberkochen (DE); O'LEARY, Joseph Allen, 73447 Oberkochen (DE); SAUR, Stefan, 73447 Oberkochen (DE)
(74) Representative: Kraus & Lederer PartGmbB

(57) **Abstract**

Various types of the disclosure pertain to determining a type of a microsurgical intervention. The type of the microsurgical intervention is determined based on metadata associated with video data encoding a microsurgical video. It is optionally possible to take into account the video data when determining the type of the microsurgical intervention.

## Description

### TECHNICAL FIELD

Various examples of the disclosure generally pertain to determining a type of a microsurgical intervention based on microsurgical video data and/or associated metadata. Various examples of the disclosure generally pertain to enabling a type-specific assessment of data associated with a microsurgical intervention.

### BACKGROUND

Medical imaging apparatuses such as operating microscopes (sometimes also referred to as surgical microscopes) employed in microsurgical interventions are oftentimes capable of acquiring microsurgical videos. In the prior art, analysis algorithms are available to analyze microsurgical videos and other data associated with the microsurgical intervention. Oftentimes, such analysis algorithms are type-specific, i.e., they are tailored to a specific type of the microsurgical intervention.

US 10,956,492 B discloses using several trained machine-learning techniques that are capable of recognizing different types of surgical procedures and, when presented with a new surgical video, classify the video for further processing. The machine-learning techniques are also capable of identifying surgical steps that may be employed in such surgical procedures. After identifying the type of surgical procedure, a second machine-learning technique that has been trained for that surgical procedure type is then used to identify the different steps taken within the video. The second machine-learning technique outputs timestamps representing when the different steps of the procedure started and ended. After receiving this information from the second trained machine-learning technique, the video processing server generates bookmarks and inserts them as metadata within the video file. Such techniques face certain restrictions and drawbacks. It has been found that determining the type of a microsurgical intervention based on a machine-learning technique alone can be comparatively inaccurate. Oftentimes, the type of the microsurgical intervention is misclassified. Also, it is difficult to train a machine-learning technique that can flexibly handle microsurgical videos across a wide spectrum of types of microsurgical interventions, e.g., across multiple specialties.

### SUMMARY

Accordingly, a need exists for advanced techniques of determining a type of surgery, in particular of a microsurgical intervention. A need exists for advanced techniques that overcome or mitigate at least some of the above-identified drawbacks and restrictions. In particular, a need exists for techniques that enable to robustly and reliably determine the type of the microsurgical intervention based on video data that encodes a microsurgical video.

Techniques are disclosed that enable to determine the type of a microsurgical intervention accurately and robustly. In particular, such determination can be used to determine the type of the microsurgical intervention across various specialties and, additionally, discriminate between different types within each specialty. For instance, it is not only possible to discriminate between cataract microsurgery and vitreo-retinal microsurgery within the specialty ophthalmology, but also discriminate such cataract microsurgery and vitreo-retinal microsurgery against microsurgical interventions in other specialties, e.g., within the field of neurosurgery, e.g., microvascular decompression, microdiscectomy, etc., to give only a few examples.

Based on such determination of the type of the microsurgical intervention, it is then possible to perform a type-specific analysis of the video data and/or of further data associated with the microsurgical intervention. Thus, the techniques disclosed herein enable postprocessing of data associated with the microsurgical intervention in a type-specific manner, tailored to the particular tasks typical for such microsurgical interventions of a given type. Based on such determination of the type of the microsurgical intervention, it is alternatively or additionally possible to perform a type-specific visualization of the video data and/or further data. Video data can be stored in a searchable structure in a data archive.

According to various examples, a multi-stage type classification can be executed. In a first stage, the type of the microsurgical intervention can be determined based on metadata that is associated with the microsurgical video or more generally the microsurgical intervention. At this first stage, the microsurgical video is not assessed. An analysis of the video data is not performed. For instance, frames of the video data are not inspected. Sometimes, it is possible to determine the type of the microsurgical intervention based on the metadata alone, i.e., without considering the frames of the video data.

Determining the type of the microsurgical intervention based on the metadata can include performing an analysis of the metadata.

In particular, it is possible to establish whether the type of the microsurgical intervention can be unambiguously determined based on the metadata. "Unambiguously" determining the type of the microsurgical intervention means that - at a certain desired granularity of the determination all other candidates in a result space can be ruled out, with the exception of a single remaining type. Unambiguously determining the type of the microsurgical intervention can be defined with respect to a certain probability level. For instance, if a given type of the microsurgical intervention can be determined at a certain probability that is larger than a predefined threshold - e.g., 95% or 99%, etc. - then it may be concluded that other types of the microsurgical intervention can be reliably ruled out. As a concrete example, if based on the meta-data, all but two types can be ruled out, and one of the remaining types has a probability of 99% whereas the second remaining type has a probability of 1% based on analyzing the meta-data, then the determination may result in selecting the first type, e.g., by assessing if the largest probability (here 99%) exceeds a given threshold (e.g., 95%).

Sometimes, it is not possible to determine the type of the microsurgical intervention based on the metadata alone without considering the video data (i.e., the type of the microsurgical intervention cannot be unambiguously determined based on the metadata and multiple types remain candidates); in such case, a second stage can follow the first stage. This means that responsive to establishing that the type of the microsurgical intervention cannot be determined based on the metadata, an analysis of the video data is performed to determine the type of the microsurgical intervention. In the second stage, it is possible to determine the type of the microsurgical intervention taking into account the video data. Thus, a relatively processing-heavy analysis of the video data is only selectively executed depending on the analysis of the metadata.

It is optionally possible to also take into account the metadata (that is previously used in the first stage) also in the second stage. To do so, the metadata may or may not be pre-processed. The metadata may be considered directly or indirectly. The metadata may be considered as an input or as a configuration. A prompt for the subsequent analysis of the video data may be determined based on the metadata. For instance, the analysis for analyzing the video data or a representation thereof in the second stage can be configured based on the metadata. Alternatively or additionally, the result space from which the analysis algorithm selects the type of the microsurgical intervention can be determined based on the metadata. A set of candidate types from which the analysis of the video data selects the type can be determined based on the metadata or a representation thereof or an analysis thereof. For instance, certain types of microsurgical interventions can be ruled out or excluded from the second stage based on the analysis of the metadata in the first stage. It would be possible to obtain probabilities of multiple types of microsurgical interventions from the first stage and take into account such information in the second stage (e.g., vitreo-retinal: 80%, cataract: 20%). The accessible result space of the second stage can be narrowed down in the first stage. As a general rule, a classification prior of the classification of the type of the microsurgical intervention in the second stage can be determined or, at least, adjusted in the first stage. The term "classification prior" corresponds to pre-existing knowledge considered in a Bayesian statistical model. In the context of a classification problem, a classification prior is a prior probability associated with the classes in the dataset (here: the different types). It is a guess or estimation about the likelihood of the occurrence of different classes before the model (here: the analysis algorithm) has seen any data (here: the video data). The classification prior can be non-probabilistic. For instance, the classification prior could - at 100% certainty - rule out certain types of microsurgical interventions. The classification prior could hard-restrict the allowable result space. It would, in some scenarios, also be possible that the classification prior is probabilistic. For instance, for a set of candidate types of microsurgical interventions, the classification prior can provide respective likelihoods. Soft constraints can thereby be imposed on a classification of the type of the microsurgical intervention executed in the second stage classification.

Various techniques are based on the finding that using such a multi-stage classification to determine the type of the microsurgical intervention allows to robustly and accurately determine the type of the microsurgical intervention even for a large accessible result space with many different types of microsurgical intervention across multiple specialties. This is especially true if compared to machine learning models according to reference implementations that are trained to predict the surgery type directly from a surgical video alone, i.e., without explicitly considering additional cues and/or application-specific knowledge about the characteristics of the surgery type.

An analysis of the video data to determine the type of the microsurgical intervention can be based on a representation of the video data. The representation of the video data can have a smaller size than the video data itself, i.e., be compressed or encoded. A representation of the video data in a latent feature space can be determined (i.e., encoding the video data using a set of underlying, non-observable variables / features). The representation of the video data can aggregate information across multiple frames. In other words, the analysis cannot be executed on a frame-by-frame basis, but rather be executed based on characteristics of the video considered across the multiple frames. This may include discarding a temporal relationship of the multiple frames of the microsurgical video. For instance, it would be possible to take into account image characteristics that are determined by statistical interpretation of information included in each frame. The representation of the video data can be based on a statistical characterization of characteristics of pixel values of at least some of the frames of the video data; then, an aggregate of such image characteristics can be considered. Alternatively or additionally, it would also be possible to determine a representation by considering semantics of the video. The representation of the video data can include an identification of a semantic content of the microsurgical video. For instance, it can be determined whether certain semantic features or patterns are present, e.g., whether certain objects are visible in one or more frames, discrimination between different types of objects, sizes of objects, locations of the objects, how often certain objects are visible throughout the multiple frames, a spatial relationship between certain objects of given type or types (e.g., do certain surgical tools appear at the same side of the frame or at opposite sides of the frames), a sequence with which certain objects are visible in the frames etc.

A computer-implemented method is disclosed. The method includes obtaining video data. The video data encodes a microsurgical video. The microsurgical video includes multiple frames. The multiple frames depict an intervention region of a microsurgical intervention. The method also includes obtaining metadata. The metadata is associated with the microsurgical intervention; for instance, the metadata may be associated with the video data. The method also includes establishing whether a type of the microsurgical intervention can be determined based on the metadata. In particular, the method can include determining whether a type of the microsurgical intervention can be unambiguously determined based on the metadata alone. The method also includes, responsive to establishing that the type of the microsurgical intervention cannot be unambiguously determined based on the metadata: performing an analysis of the video data. This enables determining the type of the microsurgical intervention. The analysis of the video data is based on the metadata.

A processing device includes a processor and a memory. The processor is configured to load program code from the memory and to execute the program code. The processor, upon loading and executing the program code, execute a method as disclosed above.

Program code that is executable by a processor is disclosed. The processor, upon loading and executing the program code, executes the method as disclosed above.

Using the techniques disclosed above, several effects can be achieved. A higher reliability of the determination of the type of microsurgical intervention can be achieved, especially when compared to a black-box machine learning model that operates on the basis of the video data, e.g. by analyzing frames. The determination result is provided with increased transparency, e.g. human understandable. The results can be explained. The above 2-stage approach can be easily extended to new types of interventions. The annotation cost for generating training data to train any machine learning models can be reduced.

It is to be understood that the features mentioned above and those yet to be explained below may be used not only in the respective combinations indicated, but also in other combinations or in isolation without departing from the scope of the invention. For illustration, above scenarios have been disclosed according to which a multi-stage type determination is executed; as well as scenarios according to which rather than assessing the video data directly, a representation of the video data is determined. Such scenarios can be used in isolation or in combination.

### BRIEF DESCRIPTION OF THE DRAWINGS

FIG. 1 schematically illustrates a system including multiple clients and a server providing interface that is accessible by users according to various examples.
FIG. 2 is a flowchart of a method according to various examples.
FIG. 3 schematically illustrates a processing pipeline for evaluation of microsurgical data according to various examples.
FIG. 4 schematically illustrates trocar detection in a frame of a microsurgical video according to various examples.

### DETAILED DESCRIPTION

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

Hereinafter, techniques of determining a type of a surgery are disclosed. Specifically, techniques of determining a type of a microsurgical intervention are disclosed. Microsurgical interventions employ operating microscopes. Some types of microsurgical interventions are listed in TAB. 1 below.

**TAB. 1: Various types of microsurgical interventions, associated with multiple specialties. According to examples, it is possible to determine the type of the microsurgical intervention across multiple specialties and by discriminating between multiple types per specialty. The candidate types from which the type of the microsurgical intervention is determined can be defined - as illustrated in TAB. 1 - based on multiple hierarchy levels, wherein the first hierarchy level is associated with different intervention regions and the second hierarchy level is associated with different types for the given intervention region of the first hierarchy level.**

| SPECIALTY | TYPE |
|---|---|
| Neurosurgery | Microvascular decompression, spine surgery, microdiscectomy, microneurosurgical clipping or coiling of cerebral aneurysms, tumor resection, treatment of arteriovenous malformations (AVMs), carotid endarterectomy |
| Ophthalmology | Cataract surgery (e.g., phacoemulsification, extracapsular lens extraction), Glaucoma surgery (e.g. glaucoma implant surgery, Trabeculectomy), Vitrectomy, Corneal transplantation, Refractive cornea surgery (e.g., LASIK, LASEK, femto-LASIK, Lenticula extraction (e.g., SMILE)), Vitreo-retinal surgery (e.g., macular hole surgery, retinal detachment surgery, epiretinal membranes peeling), Therapeutic cornea surgery. |
| | In cataract microsurgery, a small incision is made in the eye's cornea through which an ultrasonic probe is inserted to break up the clouded lens (the cataract). This process is called phacoemulsification. The fragmented lens material is then suctioned out, leaving behind the lens' elastic, transparent capsule. An artificial intraocular lens (IOL) is inserted into this capsule to replace the natural lens. The incision is often self-sealing, requiring no sutures, and the procedure is typically performed under local anesthesia. |
| | Vitreo-retinal microsurgery primarily deals with diseases affecting the retina, the macula, and the vitreous fluid in the eye. This can involve procedures such as vitrectomy, where the vitreous humor gel that fills the eye cavity is removed and replaced with a clear solution. If there's a retinal detachment, the surgeon will use small instruments to manipulate the retina back into place and then apply laser or cryotherapy to seal breaks. In the case of macular hole or epiretinal membrane, precise microsurgical techniques are used to remove the diseased tissue and restore vision. |
| | Small incision lenticule extraction (SMILE) changes the shape of the cornea to correct refractive errors. |
| Plastic and Reconstructive Surgery | Free flap procedures, Replantation of amputated digits or limbs, Nerve repairs or nerve grafting |
| Otolaryngology (ear, nose, throat, ENT) | Stapedectomy, Tympanoplasty, Microlaryngoscopy, Cochlear implant microsurgery |
| Vascular Surgery | Microvascular anastomosis, Bypass procedures |
| Orthopedics and Hand Surgery | Reconstructive surgeries involving nerves and vessels, Repair of flexor and extensor tendons |
| Dental | Root canal or periodontal surgery or dental implantation |

As will be appreciated from TAB. 1, types of microsurgical interventions can be discriminated within each of multiple specialties. Further, a result space for determining the type of the microsurgical intervention can span across multiple specialties. This means that the type of microsurgical intervention is determined across multiple specialties. Furthermore, the result space can include multiple entries per specialty. I.e., the type of the microsurgical intervention can be determined to discriminate between multiple types per specialty, but also take into account multiple specialties. More generically, the result space is wide and, furthermore, densely populated. It is possible to discriminate between microsurgical interventions in a broad spectrum, still being able to find the discriminate between microsurgical interventions of different type addressing the same intervention region. According to various examples, a robust processing pipeline to achieve this goal is presented that is able to handle microsurgical videos across multiple specialties and closely related types within each specialty. This enables to reliably determine the type of the microsurgical intervention which can then enable a type-specific analysis of data associated with the respective microsurgical intervention.

TAB. 1 illustrates example types of microsurgical procedures at a particular level of granularity. Sometimes it is possible that even higher levels of granularity for distinguishing between different types of microsurgical procedures are accessible by the techniques disclosed herein. As an example, in TAB. 1: specialty "ophthalmology", cataract surgery is mentioned as a specific type. This type can be distinguished from glaucoma and vitreo-retinal microsurgery. Such differentiation cataract - glaucoma - vitreo-retinal corresponds to one level of granularity of type determination. However, a higher level of granularity can be achieved if, for example, within cataract surgery the following different procedure types are distinguished: clear lens exchange - phacoemulsification - extracapsular cataract extraction - intracapsular cataract extraction. Similarly, on this level of procedure types, vitreo-retinal procedures can be distinguished from each other, e.g., epiretinal membrane peeling, macular hole surgery, retinal oncology, retinal tear/retinal detachment, etc. Accordingly, as will be appreciated, the level of granularity of the type determination can vary in different implementations. Sometimes, it would even be possible that a user selects the desired level of granularity. Similarly, for a given microsurgical video, the set of possible procedures can comprise procedures from different granularity levels, e.g., to distinguish cataract (i.e., without any less granular differentiation) from epiretinal membrane peeling, macular hole surgery, retinal oncology, retinal tear/retinal detachment, etc.

"Unambiguously determining a type of a microsurgical intervention" accordingly means that at a desired level of granularity all candidates but one can be ruled out. Such "ruling out" can be based on a probabilistic consideration, e.g., comparing probability levels with predefined thresholds.

Type-specific analysis algorithms are known to assess microsurgical videos acquired using an operating microscope, as well as other data associated with microsurgical interventions (e.g., state data of operating equipment, sensor data in the operating room, etc.). Example use cases of such type-specific analysis algorithms are summarized in TAB. 2.

**TAB. 2: Various examples of type-specific analysis of video data of a microsurgical intervention. Often, the analysis of the video data is relying on algorithms that are specifically trained or parametrized for handling data associated with the specific type of microsurgical intervention. It is not possible to transfer such analysis algorithm to solve similar tasks for data associated with a different type of microsurgical intervention, let alone across multiple specialties. To give a concrete example: an analysis algorithm which was trained to assess the length of phases during a cataract surgery cannot be transferred to analyze a vitreo-retinal surgery video, because the surgery phases of a cataract surgery are different from the ones of a vitreo-retinal surgery. This makes it important to be able to reliably and robustly identify the type of the microsurgical intervention before applying such type-specific analysis algorithm.**

| USE CASE | DESCRIPTION |
|---|---|
| Automated Surgical Skill Assessment / assessment of optimization potential and standardization potential | Videos of microsurgical procedures can be analyzed to assess surgical skill and technique. The microsurgical video is processed to extract relevant features. This can be used for training purposes, to provide feedback to surgeons, and to identify areas where technique might be improved. These might include the movements of the surgeon's hands or instruments, the timing of certain parts of the procedure, or specific techniques used. |
| | Key Performance Indicators (KPIs) may be determined. |
| Identification of Critical and/or Abnormal Events | Critical events during a procedure can be identified, e.g., to predict patient outcome. |
| Automated Annotation | Time-stamped annotations can be created for each significant step in the microsurgical intervention. For instance, when a surgeon makes an incision, sutures a wound, or removes a tumor, the algorithm can automatically recognize these steps and log them in the surgical record, and/or add a bookmark to the video data. |
| | Multiple phases of a microsurgical intervention can be identified. |
| Report generation | A documentation of the microsurgical intervention can be generated. The generated documentation can then be automatically integrated into the patient's electronic health record. A summary including type of the intervention, significant steps, complications, and outcome can be included in the report. |
| Visualization | Type-specific visualization, e.g., with additionally recorded modality, or with type-specific overlays. |
| | Viewing of recorded surgeries which are related, pre-selected to only show surgeries from same type |
| User error detection | The video data can be analyzed in order to determine whether a user error has occurred. Since user errors are typically specific for the type of the microsurgical intervention, the respective analysis often requires an indication of the type. |
| Localization tasks | For example, it is possible to segment or otherwise localize certain structures or objects that are specific to a certain type of microsurgical procedure, e.g., using bounding boxes or centroid localization. In order to load a domain-specific localization algorithm, it is necessary to unambiguously determine the type of microsurgical procedure depicted in the video. Semantic or instance segmentation tasks can be facilitated. |
| | For example, it would be possible to localize tools and tissue or tissue of a certain type and then conclude on the tool-tissue-interaction. Example 1: locate incision knife, locate sclera, find locations where the incision knife enters the sclera, find at least two of these locations (main incision and second paracentesis), compute the angle between them. Example 2: localize the phacoemulsification tool tip, localize the pupil, assess the relative speed of the tool tip in the pupil. |

According to various examples, it is possible to determine the type of a microsurgical intervention depicted in a video encoded by video data. This enables type-specific analysis of the video data based on one or more type-specific algorithms, e.g., as disclosed in TAB. 2.

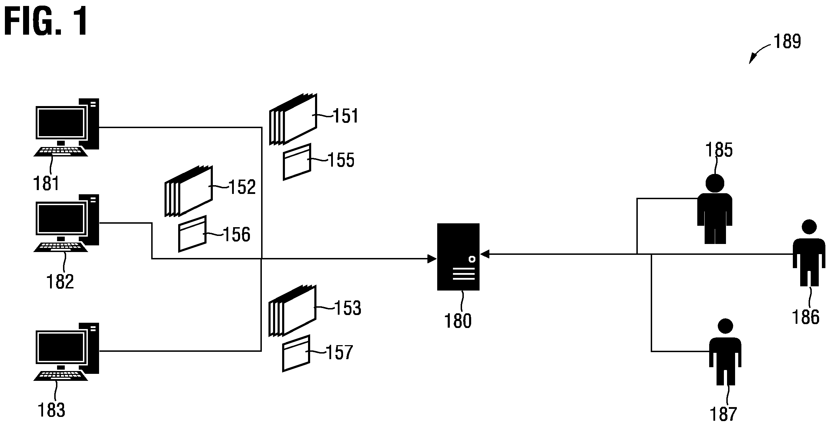

FIG. 1 schematically illustrates a system 189. The system 189 includes a server 180, e.g., deployed in the cloud or in a local network, e.g., a hospital network. The server 180 includes a processor and a memory. The processor is configured to load program code from the memory and to execute the program code. The processor, upon loading and executing the program code, performs techniques as disclosed herein, e.g., processing video data encoding a microsurgical video, determining a type of the microsurgical intervention, depending on the type of the microsurgical intervention, performing a type-specific analysis of the video data, etc.

As illustrated in FIG. 1, the server 180 obtains video data 151-153 from multiple sources 181-183, e.g., client computers. The client computers 181-183 can be coupled to or integrated into operating microscopes, more specifically to cameras thereof. The cameras can provide imaging data based on which the clients 181-183 are configured to determine the video data 151-153. The video data 151-153 is then provided / uploaded to the server 180. The video data 151-153 is associated with respective metadata 155-157.

As illustrated in FIG. 1, the server 180 can provide access to multiple users 185-187. For instance, the server 180 can provide a graphical user interface (GUI) for the users 185-187 to access the video data 151-153. A web interface can be provided. Furthermore, the GUI can enable the users 185-187 to obtain results of a type-specific analysis of the video data 151-153 (cf. TAB. 2). For this, the server 180 can be configured to determine the type of the microsurgical intervention that is depicted by each of the video data 151-153. Because the server 180 can obtain video data 151-153 from a range of clients 181-183, this implies that the imaging modalities, types of microsurgical interventions, operating settings of the operating microscope, users and surgeons vary. The techniques employed by the server 180 to determine the type of the microsurgical intervention need to be able to cope with such variability in the semantic content of the video data 151-153. Hereinafter, details with respect to such data processing implemented at the server 180 or generally at cloud-computing compute circuitry to determine the type of the microsurgical intervention will be disclosed.

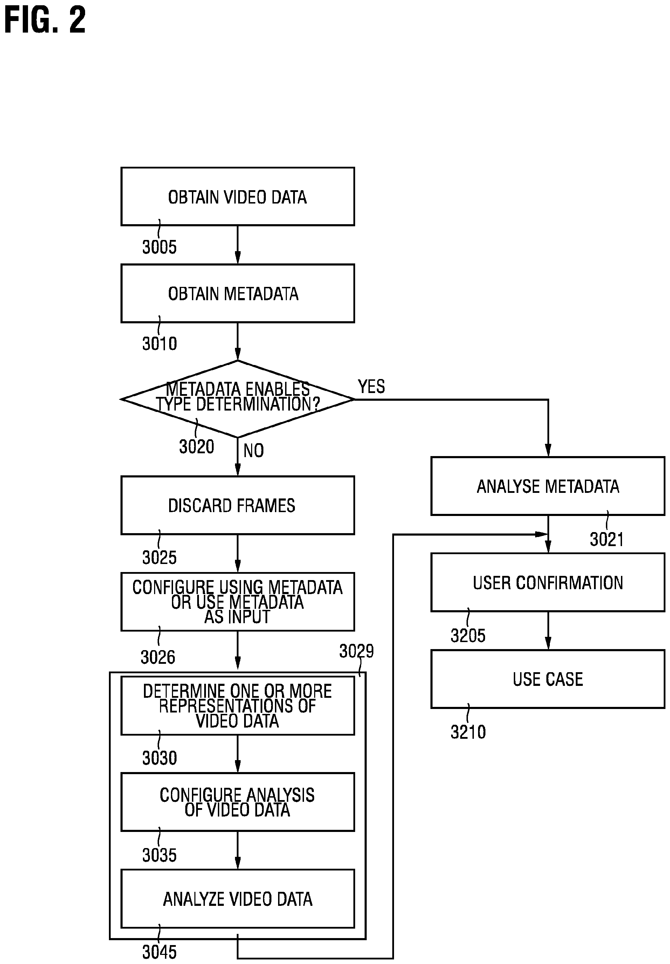

FIG. 2 is a flowchart of a method according to various examples. The method of FIG. 2 enables to determine a type of microsurgical intervention based on a microsurgical video. FIG. 2 enables to implement various use cases based on such type of the microsurgical intervention. The method of FIG. 2 can be executed by a processor. The processor can load program code from a memory and execute the program code, to then execute the method based on such execution of the program code. For example, the method of FIG. 2 can be executed by the server 180 of the system 189 illustrated in FIG. 1.

At box 3005, video data is obtained. The video data depicts a microsurgical intervention. The video data, accordingly, encodes a microsurgical video. The video includes multiple frames.

Obtaining the video data can include receiving the video data from a client computer. The client computer can be coupled to an operating microscope. The operating microscope can include a camera that acquires the video.

Obtaining the video data can include loading the video data from a repository. The repository can be stored at a server, e.g., at the Internet or at an on-premise network. The repository can be a cloud repository.

The video depicts an intervention region of a microsurgical intervention during the intervention and optionally before and after the intervention (microsurgical video). The microsurgical video is acquired using an operating microscope. The microsurgical video can cover a pre-intervention phase of the microsurgical intervention, an intervention phase of the microsurgical intervention, and a post-intervention phase of the microsurgical intervention. Accordingly, the video data can be obtained after completion of the microsurgical intervention. The video data can be uploaded after completion of the microsurgical intervention, for post-intervention review.

At box 3010, metadata is obtained.

The metadata is associated with the microsurgical intervention.

The metadata can be obtained in a similar manner as the video data at box 3005. The metadata can be linked or associated with the microsurgical video. The metadata can be embedded in the video data. The metadata can include a pointer to the video data.

The metadata can also be obtained separately from the microsurgical video.

Multiple sources of the metadata are possible and some examples are given next: (1) Operating microscope; (2) Customer Relationship Management system - e.g., cloud implemented - of surgical microscope company to match serial ID of microscope with CRM data, e.g., customer site, discipline, ... (3) HIS (hospital information system) / EHR (electronic health record), to determine the surgeon that planned for surgery etc.

The metadata can pertain to a content description relating to the microsurgical intervention. Keywords or coded information pieces can be included in respective information elements (tags). Some examples of such metadata tags are described next.

Metadata can be provided as tags associated with the image data, such as DICOM tags. Metadata can refer to additional data coming from different devices used before and/or during and/or after the microsurgical intervention. Metadata can refer to additional data derived from usage characteristics (e.g., hospital/surgeon usage information) associated with the surgical video.According to various examples, the metadata can be indicative of the medical imaging apparatus used to acquire the video data. For example, the metadata can be indicative of a type of an operating microscope used to acquire the video data. An example would be DICOM tag (0008,1090), see https-//www.dicomlibrary.com/dicom/dicom-tags/ (retrieved August 7, 2023). The metadata could be indicative of a camera type or objective type attached to the surgical microscope and used to acquire the video data. A filter setting can be indicated. A software setting can be indicated. The metadata can be associated with the medical imaging apparatus used to acquire the video data. For instance, the metadata can be indicated for predefined device configuration of the medical imaging apparatus used for acquiring images. This can include which filters are placed in the optical path during the microsurgical intervention to acquire the video data, whether a phase-contrast module is used, etc. In particular, the device confirmations can pertain to the optical configuration of the microscope and, accordingly, affect the appearance of the frames of the video. It would be possible to identify hardware modifications of the medical imaging apparatus, e.g., detected based on one or more sensors of the medical imaging apparatus or other electronic mechanisms.

The metadata may explicitly indicate the type of the microsurgical intervention. An example would be use of the DICOM tag "(0022,1040)" that identifies refractive surgery.

According to various examples, the metadata can pertain to the usage information. For instance, statistical usage of the medical imaging apparatus, e.g., resolved by users, usage time, surgeon, etc. can be provided. Such usage data can be provided in an anonymized or pseudo-anonymized manner.

According to various examples, the metadata can pertain to a software configuration of the medical imaging apparatus.

According to various examples, the metadata can pertain to a software usage pattern, e.g., which modules are loaded or used by a user.

According to various examples, the metadata can be indicative of combination of devices used for implementing the medical imaging apparatus. For instance, if a modular device system or whether a phase contrast imaging option is activated.

According to various examples, the metadata can be indicative of a user that is associated with the microsurgical intervention. For instance, a user may log into an operating system of the surgical microscope and start acquisition of the video. At this time, user credentials, e.g., a user identity, can be stored in the metadata that is generated upon acquiring the video data. Alternatively or additionally, a user may log into the repository that stores the video data to upload the video data. At this time, user credentials, e.g., a user identity can be stored along with the video data upon upload-ing the video data to the repository.

According to various examples, the metadata can, alternatively or additionally, be indicative of a location where the microsurgical intervention was performed. For instance, a deployment site of the medical imaging apparatus used to acquire the video data may be defined by a user and stored in the metadata. The deployment site can be provided at the granularity of one or more of the following: a local area network in which the medical imaging apparatus is deployed, a hospital or radiology site where the medical imaging apparatus is deployed, country or ZIP Code, latitude / longitude.

According to various examples, the metadata can be indicative of an institution where the microsurgical intervention was performed. For instance, the medical imaging apparatus used to acquire the video data can be registered to be associated with an owner. This owner can be identified by an appropriate identifier, e.g., name, credentials, etc. Then, the respective information can be provided as the metadata.

According to various examples, the metadata can be indicative of context information associated with the overall video, rather than with individual frames of the video. The metadata can be different than an amount of pixel information; e. g., in a video comprising multiple frame, the metadata can be associated with the video file, and not with the individual frames.

According to various examples, the metadata can include deterministic and/or probabilistic information. For instance, historical user data can be indicated in a probabilistic manner. This can pertain to a probability of a certain user or a surgeon - e.g., identified by a respective identity - using the medical imaging apparatus.

To give some examples: The metadata can indicate a probability of a surgeon using a certain operating microscope. The metadata can indicate a probability of a surgeon using that operating microscope to perform a certain microsurgical intervention.

For instance, the probabilistic information may be determined based on a predefined mapping that links certain microsurgical interventions to certain types of operating microscopes. This is based on the finding that certain operating microscopes are more likely to be employed at a given type of microsurgical intervention than in another type. Accordingly, if the type of the operating microscope is known, by applying the mapping certain information on the likelihood of multiple types of microsurgical interventions being executed using the operating microscope can be obtained.

According to various examples, it would be possible that the metadata is indicative of a surgery plan, e.g., by obtaining the metadata from an electronic health record or hospital information system. The operation plan can provide an overview of a mapping between surgeons and/or medical imaging apparatuses. Operating wounds can be scheduled.

The metadata can include information pertaining to one or more surgical instruments or tools (different than the operating microscope) being used in the microsurgical intervention. For instance, it would be possible that the metadata is indicative of whether a phacoemulsification device has been used. Phacoemulsification is a cataract surgery method in which the internal lens of the eye which has developed a cataract is emulsified with the tip of an ultrasonic handpiece and aspirated from the eye.

As a general rule, multiple video data stored in a repository can be associated with metadata including different information content. I.e., the metadata of a first video data can provide certain information, e.g., pertaining to the medical imaging apparatus used to acquire the video data and the institution where the microsurgical intervention was performed; while a second video data also stored in the repository may not provide such information in the metadata. Accordingly, the information content of the metadata can vary from instance to instance. Sometimes, it would even be possible that metadata is not available, at all. Accordingly, box 3010 is optional.

Techniques are disclosed that enable to flexibly employ metadata, if available, and are able to cope with the varying information content that can be conveyed by the metadata in the various scenarios.

Next, at box 3020, it is established whether a type of the microsurgical intervention can be determined based on the metadata. I.e., it can be determined whether the metadata provides sufficient information to reliably determine the type of the microsurgical intervention. Box 3020 can include determining whether the type of the microsurgical intervention can be determined unambiguously based on the metadata.

In particular, box 3020 can include determining whether - at the desired level of granularity - only a single type of microsurgical intervention is possible given the metadata.

At box 3020, the video data is not taken into account. Accordingly, it would be generally possible that box 3005 is executed after box 3020.

The check at box 3020 can operate based on pre-processed metadata. For example, if it is already checked whether certain cumulative criteria are met by a combination of several information elements of the metadata, a simple yes/no check can be implemented in box 3020. On the other hand, it is also possible that there is no or only limited pre-processing of the metadata. In such a scenario, it is possible that the decision in box 3020 requires more extensive logic.

In a first example implementation of box 3020, it would be possible to determine whether the metadata includes a tag that explicitly indicates the type of the microsurgical intervention. If available, then this type indicated by the metadata can be used as type of the microsurgical intervention.

In a second example implementation of box 3020, it would be possible to determine whether the metadata is indicative of a type of the medical imaging apparatus used for acquiring the microsurgical video. If yes, then this information can - at least sometimes - enable to determine the type of the microsurgical intervention. The medical imaging apparatus can be specifically defined for acquiring microsurgical videos of a certain type of microsurgical intervention. For instance, certain medical imaging apparatuses can be designed for eye surgery while other medical imaging apparatuses can be designed for dental surgery while still other medical imaging apparatuses can be designed for neurosurgery. Then, if the type of the microsurgical intervention is to be determined at such level of granularity (e.g., discriminating between different specialties), the metadata can suffice to make this determination. I.e., establishing whether a type of the microsurgical intervention can be determined based on the metadata can depend on a level of granularity of the type of the microsurgical intervention. For instance, for certain tasks a higher granularity distinguishing between different types of microsurgical interventions within a certain specialty may be required; while for other tasks a lower granularity can be sufficient, e.g., only discriminating between different specialties (cf. TAB. 2). As a general rule: Such level of granularity may be re-configurable. I.e., in some instances, a lower level of granularity may suffice, while higher level of granularity may be required in other instances. This may depend on the particular use case, cf. box 3210.

In a third example implementation of box 3020, establishing whether the type of the microsurgical intervention can be determined based on the metadata is based on prior knowledge. The prior knowledge can pertain to a mapping between values of information elements included in the metadata (examples have been disclosed above in connection with box 3010 and include, e.g., a medical imaging apparatus used to acquire the video data, a user associated with the microsurgical intervention, a location of the microsurgical intervention being performed, or an institution at which the microsurgical intervention is performed) on the one hand, and types of microsurgical intervention on the other hand. For instance, statistical data on the usage of a certain medical imaging apparatus in a certain institution or location and/or by a certain surgeon and the associated type of microsurgical intervention can be available. The prior knowledge can be based on historical information on usage patterns. The prior knowledge, in other words, can enable to determine the type of the microsurgical intervention if statistical data or historical information is available.

In a fourth example, the metadata includes information regarding the type of the microscope used for acquiring the video data. Then, there may be a predefined mapping that maps different types of the microscope to candidate types of the microsurgical intervention. This mapping may or may not be probabilistic. For instance, a probabilistic mapping may map a given type of a microscope to multiple candidate types of the microsurgical intervention, wherein each of the mapped candidate types of the microsurgical intervention is attributed a respective probability. Such mapping may be continuously adapted, e.g., each time ground truth becomes available.

In a fifth example, a specific microscope type indicates ophthalmic specialty. If a microscope tilt of the specific microscope type was used during surgery (the tilt being indicated by the metadata), the glaucoma type of microsurgical intervention is present.

In a sixth example, a particular type of microscope indicates a neuro specialty. If a particular infrared filter is used (e.g., cutoff at 800 nm), vascular microsurgery will be observed. If, on the other hand, a blue filter is used - e.g., with a cut-off wavelength of 400 nm - then a tumor microsurgery is present. On the other hand, if a yellow color filter is used, several candidate types of microsurgical intervention are possible, with no clear determination possible at this stage. Such examples as presented above can also be combined with each other, to form further examples.

If it is possible to determine the type of the microsurgical intervention based on the metadata, the method commences at box 3021. At box 3021, the type of the microsurgical intervention is determined based on the metadata. Typically, since the check at box 3020 already requires an analysis of the metadata, box 3021 can be limited to merely using the result of box 3020 as the final result for the type classification. In other examples, it would also be possible that the metadata is further analyzed at box 3021. To give a concrete example, it may be checked at box 3020 whether the type of the operating microscope equates to a certain value. If this is true, then the check at box 3020 may be positive - because it is known that when the type of the operating microscope has this value, it is possible to unambiguously determine the type of the microsurgical intervention based on the metadata alone. A further assessment of the metadata, e.g., of an information element that describes the tilt of the operating microscope, can then be performed at box 3021, to finally discriminate between different remaining types of the microsurgical intervention.

Then, the method commences at box 3205. Details with respect to box 3205 will be explained later; first, a scenario will be discussed in which it is not possible to determine the type of the microsurgical intervention based on the metadata alone. In such scenario, the method commences at box 3025.

At box 3025, it is optionally possible to discard at least some of the frames of the video. Discarding frames of the video means that discarded frames are not taken into account in the subsequent analysis of the video data.

Discarding frames can include removing or permanently deleting such frames from the video data. Discarding frames can also include labeling such frames as discarded so that, subsequently, they are not taken into account when analyzing the video data. Then, the frames may be retained in the video data so that any postprocessing of the video data can still consider the frames, if required.

Discarding frames can also mean creating a copied version of the video data where only specific frames are selected and using this copied version subsequently.

Discarding frames can also mean creating an index-vector which contains information about which frames to consider and/or which frames to discard and using this index-vector subsequently when analyzing the video data.

In particular, it is possible to discard such frames that carry no or only limited or redundant information that would enable to determine the type of the intervention. It would also be possible to discard frames based on image properties. For instance, it would be possible to use a focal measure to identify black or blurry frames that are not carrying significant information due to respective image artifacts. Alternatively or additionally, it is possible to discard frames of the video that are associated with a pre-intervention phase or a post-intervention phase of the microsurgical intervention. In the pre-intervention phase the microsurgical intervention itself has not yet commenced. I.e., the surgeon has not yet interacted with the tissue of the patient. For instance, the surgical team can prepare the operating room and place the patient on the operating table during the pre-intervention phase. The medical imaging apparatus, e.g., the operating microscope, may be prepared for the subsequent intervention phase so that the operating system is started and the video acquisition already commences. However, at this time, the patient may not yet be placed on the patient's table or if the patient is placed on the operating table the interaction of the surgeon with the tissue of the patient may not yet have commenced. Similarly, in the post-intervention phase the patient may not be placed on the operating table, anymore. I.e., the intervention region may not be visible in the video, anymore. In the post-intervention phase it is possible that the video acquisition is still ongoing, however, interaction of the surgeon with the tissue of the patient has already been completed. Accordingly, in the pre-intervention phase and the post-intervention phase, the amount of information included in the video is limited so that it is difficult or not possible to reliably determine the type of the microsurgical intervention based on the frames of the pre-intervention phase and the post-intervention phase. By discarding such frames, it is ensured that the subsequent analysis does not draw unreliable or false conclusions based on such frames of the video.

Various options are available for identifying frames that are to be discarded, e.g., because they are associated with the pre-intervention phase or the post-intervention phase. For instance, such frames may be manually labeled by the user. The user may manually select and discard these frames. It would also be possible that corresponding information is included in the metadata, e.g., by respective tags that label associated frames with one of the following phases: pre-intervention phase, post-intervention phase, intervention phase. In another scenario, the frames of the video can be analyzed to determine the particular phase of the microsurgical intervention associated with each frame. For instance, a cluster analysis can be performed, wherein the cluster analysis pertains to statistical characterization of characteristics of pixel values of at least some frames of the video data. For instance, it would be possible to determine histograms of the brightness values of the pixels of each frame of the video. Then, a cluster analysis of clusters formed by these distributions of the brightness values can be considered. Typically, the brightness can change between the pre-intervention phase in the intervention phase, because surgical illumination is only switched on during the intervention phase. Then, different clusters are formed by the pixel value brightness histograms associated with frames in the pre-intervention phase and post-intervention phase on the one hand, and the intervention phase on the other hand. One specific implementation of box 3025 includes applying a DBSCAN clustering algorithm. Video statistics are computed based on the maximal frame cluster, where frames are clustered based on the similarity in their cumulative pixel intensity histograms. Another option includes spatial-temporal clustering which considers the temporal proximity of each frame in addition to the intensity histograms.

Box 3025 is optional. In some scenarios, it would be possible that the video data is preprepared so that it only includes frames carrying relevant information for determining the type of the microsurgical intervention.

It would also be possible to discard frames of the video based on a sub-sampling scheme. For instance, every n-th frame may be selected in all non-selected frames may be discarded. For instance, it would be possible to keep six frames per minute and discard all other frames. It would also be possible to retain key frames that are representative for temporal clusters / phases. It would also be possible to retain or discard frames that are similar to a certain set of reference frames.

At box 3029, the type of the microsurgical intervention is determined by performing an analysis of the video data. This is responsive to establishing at box 3020 that the type of the microsurgical intervention cannot be determined based on the metadata alone. Nonetheless, as a general rule, even if the metadata is not suitable for unambiguously determining the type of the microsurgical intervention, it is still possible to take into account the metadata when performing the analysis of the video data at box 3029. The metadata (possibly pre-processed) can serve as a prior of and/or an additional input to the type classification executed subsequently at box 3029. This is illustrated in optional box 3026.

The metadata can provide additional information that enables to determine the type of the microsurgical intervention more reliably. As a general rule, the analysis of the video data executed at box 3029 is based on the metadata, i.e., can use at least a part of the metadata or a representation of the metadata as an input. Alternatively or additionally, the analysis of the video data that is executed at box 3029 can be configured based on the metadata.

For instance, certain types of microsurgical interventions can be ruled out by considering the metadata. A filtering of candidate types can be performed based on the metadata. The set of candidate types of the microsurgical intervention from which the actual type is to be selected by the analysis performed at box 3029 can be determined based on the metadata. The result space for the analysis at box 3029 can be narrowed down based on the metadata. For instance, a distance-based classifier, e.g., a nearest neighbor classifier, can be used and the elements to be considered for the distance evaluation can be restricted to elements which are consistent with the one or more candidate types obtained from the metadata, e.g., only to microsurgical videos which are from one or more of the one or more candidate types.

Such techniques can be based on using a classification prior that is determined based on the metadata. I.e., the task of classifying the microsurgical procedure (to obtain the type thereof) can be solved using information derived from the metadata as prior.

For example, an analysis algorithm for performing the analysis of the video data can be selected based on the metadata (so-called "model selection"). For instance, the analysis algorithm can be selected depending on one more candidate types of the microsurgical intervention that are determined based on the metadata, and/or a count of the one or more candidate types of the microsurgical intervention determined based on the metadata.

To give a concrete example: It would be possible that the metadata includes a tag that enables to determine the specialty of the microsurgical intervention. For instance, the metadata may carry information that allows to conclude that an ophthalmology microsurgical intervention is depicted by the microsurgical video. The particular type of the microsurgical intervention within this specialty (cf. TAB. 1: second row) may not be unambiguously determinable based on the metadata alone. However, an appropriate analysis algorithm for analyzing the metadata can be selected that is specifically tailored to discriminate between different ophthalmology microsurgical interventions, e.g., between cataract microsurgery and vitreo-retinal microsurgery. A different analysis algorithm for analyzing the video data may be selected if it is possible to determine based on the metadata that a neuro-microsurgical intervention is depicted in the video. As will be appreciated, the metadata can therefore serve as a prior to this classification task that enables to tailor the analysis of the video data, thereby yielding more accurate results.

In a further example, it is possible to select different analysis algorithms depending on whether the candidate types span multiple specialties or are limited to a single specialty. For example, a generic algorithm may be selected to analyze the video data of a large number of candidate types of microsurgical procedures obtained as a classification prior from the analysis of the metadata (e.g., because the metadata contains little information). Such a generic algorithm may not be limited to a very specific task, but may be configurable to solve different tasks in different domains. It may be of high complexity. I.e., instead of selecting a (specific) analysis algorithm, it is also possible to select a configuration for a (generic) analysis algorithm. As an example, it is possible to select a text prompt, i.e., a textual description of an intended analysis, which serves as a configuration for a multi-modal foundation model that is capable to process multiple modalities such as text, video, and/or image.

In another scenario, based on the metadata, it is possible to significantly limit the result space, e.g., to closely related types of microsurgical procedures within a single specialty. In such a scenario, a different, less complex and domain-specific analysis algorithm can be selected. To give a concrete example, it would be possible to select a domain-specific analysis algorithm to detect trocars in frames of a microsurgical video that is confirmed to represent the eye in an ophthalmic procedure.

When selecting the appropriate algorithm, it is also possible to take into account probabilistic classification priors. For instance, if - based on the metadata - it is not possible to determine the specialty at the certain minimum probability level, then a generic analysis algorithm may be selected, rather than a domain-specific analysis algorithm.

Sometimes, it is possible to select multiple algorithms. For instance, if probabilistic classification priors indicate multiple types with high likelihood, then multiple analysis algorithms tailored to process the respective types indicated to have a high likelihood can be selected. Here, it can be advantageous if these analysis algorithms also output a reliability measure to avoid out-of-distribution processing with falsified results to be considered in the downstream process.

At box 3030 it is optionally possible to determine a representation of the video data. Then, the subsequent analysis of the video data can be performed based on the representation of the video data.

The representation of the video data can be tailored to the task of type classification. Accordingly, non-relevant information may be discarded. The representation may have a relatively smaller file size than the video data, thereby enabling fast and resource efficient processing and storing. Also, the ability for manual verification and/or assessment may be improved due to aggregated information.

The representation can be on video level. I.e., the representation can aggregate information across the multiple (potentially non-discarded) frames of the video data. This means that rather than performing a frame-by-frame analysis of the video data, a global analysis of the video data based on aggregated information across all of the relevant frames can be performed. These techniques are based on the finding that the type of the microsurgical intervention can be reliably determined based on such a global representation of the video without the necessity of analyzing individual frames. Thus, the analysis of the video data is not an image-to-type analysis. For instance, different types of surgery are known to have different brightness profiles than others. To give a concrete example, it would be possible to extract pixel intensity histograms from all frames as frame representations. Then, these pixel intensity histograms can be fused into a single representation for the entire video, by averaging all frame representations. It would then be possible to discard some of these entries of the fused frame representation and rely only on certain remaining histogram bins. For instance, has been found that by relying only on a fraction of the fused histogram pertaining to the bright pixels, more robust determination of the type can be achieved, because it is those bright pixels that carry the relevant information pertaining to the type of the microsurgical intervention. In such scenario, as will be appreciated from the above, the representation of the video data also discards a temporal relationship of the multiple frames. This is because the average of the information aggregate across the multiple frames, in the above example the average of the pixel brightness histograms, is invariant against the sequence of the frames being changed. It has been found that type classification of the microsurgical intervention can be particularly robust if such temporal information is discarded. This is because sometimes the temporal order of certain steps of the microsurgical intervention is dependent on the particular surgeon and even a particular surgeon can sometimes execute steps in a different order. To enable robustness against such instance-specific sequences of actions, the temporal invariant representation of the video data can be preferable in some scenarios. However, it is not required in all scenarios to discard a temporal relationship of the multiple frames. For instance, it would be possible that an average of pixel intensity histograms is calculated in a weighted manner so that frames in the middle of the video are impacting the aggregated pixel intensity histogram more than frames at the beginning and the end of the video. Here, some temporal relationship is maintained in an implicit manner in the aggregated pixel intensity histogram. Similar examples also applied to other statistical characterizations and characteristics of pixel values.

As a general rule, as illustrated by the example provided above, the representation of the video data can be based on a statistical characterization of characteristics of pixel values of the frames of the video data. This could be labeled image characteristics that are determined by a statistical interpretation of information included in the video data.

The examples are not limited to the above-discussed histograms of pixel brightness values. Rather, other characteristics can be considered beyond brightness, e.g.: contrast, color, gray value, gradient count, edge count, etc.

Also, the statistical characterization is not limited to histograms. Other examples would be possible, e.g., averages, medians, etc. The statistical characterization may or may not be implemented as an aggregate across the characteristics of the pixel values of the frames. The statistical characterization can be limited in accordance with a predefined range of the characteristics of the pixel values. For instance, as explained above, only a certain range of histogram bins of a pixel brightness histogram can be taken into account. Thereby, less relevant or ambiguous ranges of the characteristics of the pixel values can be omitted from the analysis, thereby increasing the robustness against false determination of the type of the microsurgical intervention.

Next, a few further concrete examples of such statistical characterization of pixel value characteristics are given: First, entropy of the video average pixel intensity histogram. Second, argmax pixel intensity value. Third, median pixel intensity. Fourth, 70-th percentile pixel intensity. Fifth, width (in pixel value units) of the central 68th percentile pixel intensity. Sixth, maximum (normalized) pixel intensity. Seventh, number of peaks in average pixel intensity histogram. Eighth, frame-wise pixel intensity entropy sampled at 1% video intervals. Ninth, one or more of the aforementioned, but only with one or more selected color channels, e.g., red channel only. Tenth, cumulative pixel intensity histogram.

Alternatively or additionally to such statistical characterization, it would also be possible to employ a machine-learning algorithm to determine the representation. For instance, an encoder can be applied to determine an encoded representation (sometimes also referred to as representation in latent feature space). This may include applying a machine-learned encoder branch; it may be trained using an autoencoder network and self-supervised learning techniques. One or more convolutional layers and/or attention layers may be used. An encoder can be applied to determine an encoded representation (sometimes also referred to as representation in latent feature space). This may include applying a machine-learned encoder branch; it may be trained using an autoencoder network and self-supervised learning techniques. In further detail: The encoder branch of an autoencoder network takes an input vector (here, defined by the video data, e.g., including the pixels or pixel patches as entries of the vector) and maps it to a smaller, denser representation, called the "latent space" or "encoded representation". This process may involve using a series of dense, convolutional, and/or attention layers, followed by activation functions like ReLU, sigmoid, softmax, and/or normalization layers like group normalization or layer normalization and/or residual connections. For training, the autoencoder uses the original input vector as the target output. The model is trained to minimize the difference, often measured by mean squared error or binary cross entropy, between the original input and the output from the decoder branch. This is a self-supervised learning technique because it does not rely on labeled data, e.g., provided by an expert using manual annotation; instead, it learns to reconstruct its own (optionally masked) input. During training, the model learns to identify the most important features in the data, such that the encoded representation can be used to recreate the original input with minimal loss.

It is also possible to determine a representation of the video data based on distinct features, i.e., based on an identification of a semantic content of the video. For instance, it would be possible to determine whether the one or more objects of a pre-defined type are present in the video. A count of such objects of a predefined type in the video can be determined. A duration of a visibility of objects of predefined types in the video can be determined. A sequence of appearance of one or more objects of predefined types in the video can be determined. These are all examples of how a semantic analysis of the video can be performed.

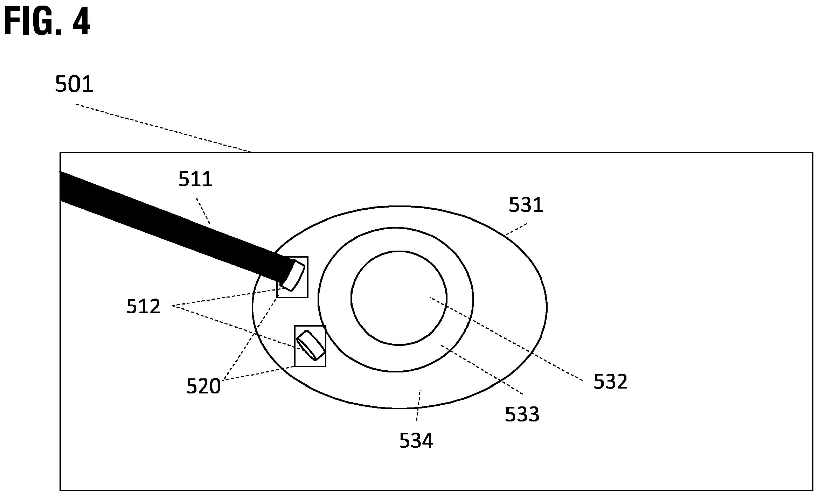

To give a concrete example: it would be possible to determine whether a certain type of surgical instrument, e.g., a trocar, is present. It would be possible to determine the count of trocars visible. A respective example implementation will be discussed later on in FIG. 4.

Such techniques are based on the finding that different types of microsurgical interventions can often times be discriminated based on the objects that are visible in the microsurgical video. For instance, certain characteristic medical instruments and/or surgical tools can be visible in the microsurgical video. To give a few examples, often times aneurysm clips are specifically used in neurosurgical interventions. Specific lens fragmentation instruments (e.g., phacoemulsification devices) are used in cataract surgery. Tissue transfer instruments are specifically used in plastic and reconstructive surgery. In a further example, trocars are used in vitreo-retinal eye surgery (a detailed example will be explained in connection with FIG. 4 later on).

Objects or other semantic content can be recognized and optionally localized (e.g., segmentation or bounding box localization) using prior-art techniques such as convolutional neural networks or as a frame-based image processing algorithms. It would be possible to take into account an optical flow between adjacent subframes in order to separate foreground from background. It is not required in all scenarios that a particular object is localized in a frame; rather, it can be sufficient to determine whether a certain object of a given type is visible anywhere in a frame.

Object recognition or localization algorithms can sometimes operate on individual frames of the video data. Object recognition or localization algorithms may also enforce consistency between recognition and/or localization results of successive frames. For example, due to image noise or temporal occlusion, certain objects may not be visible and/or reliably detectable in certain frames. However, by considering the consistency between successive frames, a reliable prediction can be made whether certain objects are located at a certain position or at least present in a frame.

In some scenarios, the semantic content of the video may also be characterized by imaging properties or presence of image artifacts. For instance, based on the metadata, it can be concluded the specialty is "ophthalmology". However, the type of the microsurgical intervention (within this specialty) may not be derivable from the metadata alone. Thus, in the second stage, the video data is analyzed for certain artifacts: if a specific visualization pattern with vignetting and a dominant red channel is observed, vitreo-retinal microsurgery is present. On the other hand, if a fluorescein-specific visualization is observed with dark blue and light green dominant, several corneal and retinal types are candidates.

At box 3035, it is optionally possible to configure the analysis of the video data. This configuration can be based on one or more decision criteria different than the metadata (As mentioned above, the analysis of the video data can be alternatively or additionally also configured based on the metadata in box 3026.). For example, it would be possible to select an analysis algorithm for performing the analysis of the video data. For example, such configuration can depend on one or more characteristics of the representation of the video data that as determined at box 3030. To give an example, it would be possible that if certain types of surgical tools are visible in the video data - this can be determined based on an analysis of the semantic content of the video - a certain analysis algorithm is selected. For instance, it would be possible to select between a probabilistic analysis algorithm for determining the type of the microsurgical intervention and a non-probabilistic rule set analysis algorithm. To give an example, if a certain microsurgical tool is visible in the microsurgical video, then a non-probabilistic ruleset analysis algorithm may be used in order to discriminate between the respective candidate types. Such non-probabilistic ruleset analysis algorithm may, e.g., take into account a sequence or timing with which the respective microsurgical tool appears in the microsurgical video. For instance, if the microsurgical tool of the given type appears before another microsurgical tool of another type, then a microsurgical intervention of a first type is selected as the result; on the other hand, if the microsurgical tool of the given type appears after the another microsurgical tool of the another type, then a second type is selected. An If-This-Then-That-type selection (or more complex derivatives thereof) can be employed. Multiple linked decision criteria can be used. In such examples, the outcome of the ruleset analysis algorithm is non-probabilistic, i.e., definitive without an associated probability. This is different for a probabilistic analysis algorithm, e.g., a machine-learned classification algorithm. Such machine-learned classification algorithm outputs a set of probabilities and/or scores associated with the different types of microsurgical interventions.

At box 3045, the video data is analyzed. This is based on applying a certain analysis algorithm to one or more representations of the video data or the video data directly.

The analysis algorithm can be selected at box 3035 (i.e., based on a representation of the video data) and/or at box 3026 (i.e., based on the metadata ).

The analysis can be performed by applying a machine-learning classification algorithm. Some example options for a machine-learning classification algorithm are described next. First, Logistic Regression: This is a supervised learning algorithm used for binary classification problems. It uses the logistic function to model a binary dependent variable, predicting the probability of the occurrence of an event by fitting data to a logit function. Second, support Vector Machines (SVM): This algorithm creates a hyperplane or set of hyperplanes in a high-dimensional space, which can be used for classification or regression. The optimal hyperplane is chosen in such a way that it has the largest distance to the nearest training data points of any class, maximizing the margin. Third, Random Forests: A random forest operates by constructing multiple decision trees at training time and outputting the class that is the mode of the classes of the individual trees. This reduces the risk of overfitting and improves the model's generalizability. Fourth, a deep neural network: data is passed through a series of interconnected layers, each composed of nodes or 'neurons', that transform the data progressively to make a final prediction. The input layer receives the data, the output layer makes the final prediction, and the hidden layers perform computations in between. In a classification task, the final output layer typically uses a softmax function for multi-class problems, which assigns a probability for each category that sums up to one, or a sigmoid function for binary classification problems, which returns a probability between 0 and 1 indicating how likely the sample belongs to a certain class.

Such machine-learning algorithms can be trained based on manually annotated video data. Domain experts can provide respective labels as annotation and then it is possible to use respective representations of the video data in combination with the ground truth in form of the labeled annotations to minimize a loss of the respective machine-learning algorithm. As a general rule, it would be possible to train the machine-learning algorithm, specifically if implemented as a deep neural network, simultaneously with another task, e.g., phase segmentation, in a multitask manner.

Thereby, one decoding branch can predict the phase of the microsurgical intervention and another branch can predict the type of the microsurgical intervention.

To give a concrete example for a machine-learning algorithm operating based on a representation of the video data: it would be possible to use the first 40 bins of an aggregated pixel brightness value histogram (i.e., the first 40 bins corresponding to the bright part of the histogram). A respective vector is then specified using a random forest classifier to discriminate between two types of microsurgical interventions, namely cataract microsurgical intervention and vitreo-retinal microsurgical intervention. For instance, good results have been obtained by a training based on 50 cataract videos and 50 vitreo-retinal videos. Robustness of the feature/model can be validated through K-fold cross validation on the limited subset of 100 training videos and using stratified K-fold cross validation of the complete collection of cataract videos and vitreo-retinal videos. The random forest machine-learning classification algorithm is a comparatively thin machine-learning model that provides robust results. The random forest machine-learning classification algorithm can be trained with limited data and is computationally efficient. For instance, graphics processing unit (GPU) accelerators are not typically required.

It is also possible to employ one or more predefined decision-making rule sets in box 3045. For instance, it would be possible to determine, using a machine-learning algorithm, whether certain surgical tools are visible in one or frames of the microsurgical video (at box 3030). Then, at box 3045, it is possible to use the decision-making rule set to determine the type of the video based on respective information regarding the presence of certain microsurgical tools of multiple types in the microsurgical video.

Some examples are provided below:
(i) More than two trocars detected in N subsequent frames (N>30), then the analysis result will be vitreo-retinal microsurgical intervention.
(ii) cornea incision knives detected AND absence of rhexis tools AND absence of phacoemulsification and irrigation/aspiration tools AND (presence of a phakic IOL injector OR phakic IOL manipulator), then the analysis result will be phakic intra-ocular lens implementation ELSE glaucoma stent surgery

Beyond such non-probabilistic decision-making rulesets, it would be generally possible to consider probabilistic decision-making rulesets. It is also possible that several indicators serving as input to such decision-making ruleset are present, but even their joint consideration does not allow a 100% statement. In general, each indicator can provide a probability distribution over the possible types of microsurgical interventions. The joint consideration can then lead to a final probability assessment. The prior probability distribution can also be taken into account.

As will be appreciated from the above, multi-dimensional and/or multi-hierarchy decision-making, e.g., using decision trees reflecting a certain hierarchy between different decisions, can be implemented based on the representation of the video data.

As a general rule, such classification algorithms to determine the type of the microsurgical intervention at box 3045 can operate not only based on an input that corresponds to the representation of the video data (as obtained from box 3030). Additionally, such classification algorithms can also obtain an input that is based on the metadata. Respective techniques have been discussed briefly in connection with box 3026 above.

Once the type of the microsurgical intervention has been determined (either at box 3021 based on the metadata alone, or at box 3045 based on the video data and possibly taking into account the metadata), the method commences at box 3205.

At optional box 3205, it is possible to obtain user confirmation of the determined type. For example, it would be possible to determine a reliability with which the type of the microsurgical intervention has been determined. Then, depending on the reliability, the user confirmation at box 3205 can be selectively obtained. For instance, user confirmation may only be obtained where a probabilistic analysis algorithm is employed at box 3045; user confirmation may not be required if the type of the microsurgical intervention can be determined based on the metadata alone at box 3020.

Then, at box 3210, a use case can be implemented. The use case makes use of the type of the microsurgical intervention previously determined. Various post-processing use cases have been previously discussed in connection with TAB. 2.

For instance, an analog or digital trigger signal can be provided to downstream processing circuitry to implement the use case. The trigger signal can be indicative / depend on the type of the microsurgical intervention. For instance, one or more machines can be controlled depending on the type. For instance, feedback can be provided to the user depending on the type. It would be possible to output a classification of the type of the microsurgical intervention to a user, e.g., via a GUI platform. For instance, the video data can be tagged with the respective type of the microsurgical intervention. This enables to make the repository that stores the video data searchable. It would be possible that the type of the microsurgical intervention is appended to the metadata that is stored in association with the video data in the repository; a new tag can be created. The metadata may or may not be embedded into the video data. The metadata may include a link or pointer to the video data. It would be possible to execute one or more type-specific analysis algorithms based on the determined type of the microsurgical intervention. I.e., a further analysis of the microsurgical intervention can be implemented. Such type-specific analysis algorithm that is executed above 3210 can also operate based on the video data. For instance, a frame-by-frame analysis would be conceivable. The particular type-specific analysis algorithm that is employed at box 3210 can be selected from a repository of analysis algorithms based on the type that has been determined as disclosed above. For instance, non-cataract cases should not be wrongly analyzed using a cataract-only analysis algorithm. For instance, the vitreo-retinal cases can load specific visualiza-tions which are not appropriate for cataract cases, to give an example.

Summarizing, the method of FIG. 2 enables to reliably determine the type of a microsurgical intervention. The method can be modified in scenarios, deviating from the explicit example of FIG. 2. For instance, above a scenario has been disclosed in which the video data is obtained at box 3005 that is executed prior to box 3020; however, it would be possible that the video data is only selectively obtained upon determining that the metadata is insufficient for unambiguously determining the type of the microsurgical intervention at box 3020. In other words, box 3005 can be executed after box 3020. To give a further example, above scenarios have been disclosed in which a single representation of the video data is determined at box 3030 and then used as an input to the analysis of the video data at box 3045. However, according to various examples, it would be possible to determine multiple representations of the video data and use these multiple representations as input to the analysis algorithm at box 3045, e.g., in a concatenated manner. For example, it would be possible to determine a first representation based on a statistical characterization of characteristics of pixel values of at least some frames of the video data and, furthermore, determine a second representation depending on the semantic content of the video.

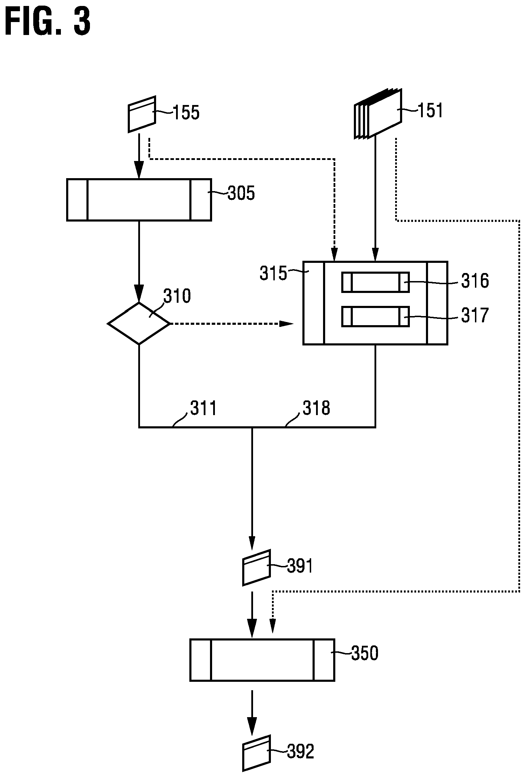

FIG. 3 schematically illustrates a processing pipeline for evaluating data associated with a microsurgical intervention. The processing pipeline can implement the method of FIG. 2. In FIG. 3, the processing pipeline operates based on the microsurgical video encoded by the video data 151 and the associated metadata 155. FIG. 3, in particular, illustrates multiple ways to take into account the metadata 155 when determining the type of the microsurgical intervention.

Provided is an analysis algorithm 305 that processes the metadata 155 (this is optional). At decision point 310, it can be assessed whether the microsurgical intervention can be determined unambiguously based on the metadata 155 (cf. FIG. 2: box 3020). This may be based on an output of the analysis algorithm 305. In other words, it may be checked whether the class distribution is unambiguously indicative of a single type of microsurgical intervention.

If it is established that the metadata 155 allows to unambiguously determine the type of the microsurgical intervention, the output 391 (an indicator indicative of the type; this implements box 3021 in FIG. 2) is provided based on the metadata 155 or more specifically the output of the analysis algorithm 305, if available (branch 311).

Otherwise, another analysis algorithm 315 is used to determine the output 391, i.e., to classify the type of the microsurgical intervention (branch 318). The analysis algorithm 315 generally corresponds to box 3029 of the method of FIG. 2. The analysis algorithm 315 obtains, as an input, the video data 151.

It is possible that the analysis algorithm 315 includes a module 316 that determines a representation of the video data 151, as previously explained in connection with FIG. 2: box 3030. Furthermore, the analysis algorithm 315 includes a module 317 that determines the output 391, e.g., based on the representation of the video data 151 as obtained from the module 316, if available. The module 316 and/or the module 317 can obtain, as a further input, the metadata 155 and/or a representation thereof. The module 316 and/or the module 317 can obtain, as a further input, a class distribution prior determined based on the metadata. For instance, a set of candidate types of the microsurgical intervention as obtained from the analysis algorithm 305 can be provided to the analysis algorithm 315 for final selection of the actual type of the microsurgical intervention. For instance, the algorithm for implementing module 317 can be selected based on the output of the analysis algorithm 305 or directly based on the metadata 155. It would also be possible that the module 317 uses, as a further input, e.g., concatenated with the representation of the video data, the output of the analysis algorithm 305.

The output 391, i.e., the indication of the type of the microsurgical intervention, can then be used in a type-specific analysis algorithm 350, to determine an output 392 associated with the microsurgical intervention. The output 392 could pertain to, e.g., one or more of bookmarks for the video data, a medical report drawn up for the microsurgical intervention, a trigger signal, an identification of abnormalities, a quality rating, a patient outcome indicator, etc. Also see TAB. 2 above. The analysis algorithm 350 can obtain, as an input, the video data 151 or other data associated with the microsurgical intervention (not shown in FIG. 3). The particular implementation of the type-specific analysis algorithm 350 is not germane for the techniques discussed herein. Prior art techniques can be employed. See, e.g., WO2023 021074 A1.

Next, a possible implementation of the method of FIG. 2 and of the processing pipeline of FIG. 3 as discussed in connection with FIG. 4.

FIG. 4 illustrates a frame 501 of a microsurgical video. Depicted is the eyeball 531, the pupil 532, the iris 533, and the sclera 534. Also visible are trocars 512 and a microsurgical instrument 511. A trocar is a sharp, pen-like instrument used to create an entry point into the eye. Typically, two or three trocars are used in vitreo-retinal surgeries, such as vitrectomies, to create three ports in the sclera (the white part of the eye). These ports serve as entry and exit points for various surgical instruments and fluids.

Next, a concrete implementation of the processing of video data and metadata is provided to determine the type of microsurgical intervention as vitreo-retinal.

First, the metadata is analyzed. The metadata includes an information element that is indicative of a particular type of operating microscope that is specifically adapted to perform an eye surgery. Accordingly, it is not possible to uniquely determine the microsurgical procedure based on the metadata alone (FIG. 2: box 3020 - "no" path). The method then proceeds to determine the type of microsurgical intervention based on an analysis of the video data. This analysis of the video data can use as a prior the fact that the type of microscope is specifically adapted to support the ophthalmic surgery. In other words, the candidates for video data analysis can be limited to microsurgical interventions in the field of ophthalmology (see TAB. 1). In particular, these include some types of microsurgery that manipulate the anterior segment of the eye and some types of microsurgery that manipulate the posterior segment of the eye. In order to distinguish between, for example, a cataract surgery that manipulates the anterior segment of the eye and a vitreo-retinal microsurgery that manipulates the posterior segment of the eye, an analysis algorithm that detects trocars can be selected. Thus, based on the analysis of the metadata, the appropriate analysis algorithm (here: trocar localization) is loaded. As shown in FIG. 4, two bounding boxes 520 are illustrated, which are determined by such a trocar localization and analysis algorithm. These bounding boxes delineate the location of trocars 512. Based on the finding that there are trocars depicted in frame 501, it is possible to conclude that the type of microsurgical intervention is: vitreo-retinal. In a practical implementation, it could be determined whether at least two trocars are continuously detected in at least N subsequent frames, where, e.g., N > 30. For example, reliable trocar detection results have been obtained with an R-CNN object detection algorithm trained on 500 frame-ground-truth pairs. Such techniques are generally known in the prior art, see, e.g., Lee, Jiann-Der, et al. "Automatic Surgical Instrument Recognition-A Case of Comparison Study between the Faster R-CNN, Mask R-CNN, and Single-Shot Multi-Box Detectors." Applied Sciences 11.17 (2021): 8097.

Since the respective object detection algorithm is only loaded in response to the determination that the frames of the microsurgical video depict an eye surgery (this determination is made based on the metadata), the object detection algorithm can be comparatively simple and requires only a limited number of training iterations. The object detection algorithm can be domain specific. It does not have to be transferable to other domains. Thus, a robust and accurate result can be achieved by implementing a two-step approach (first, restricting the candidates of the type of microsurgical intervention to the specialty of ophthalmology, and then, second, applying an appropriate domain-specific analysis algorithm based on the image data). The classification result is robust and transparent, since the final decision is based on a semantic feature which is previously extracted (here: the detection of trocars). Thereby, prior knowledge of necessary/indicative semantic features for the presence of a specific surgery type are explicitly considered, which renders the analysis step robust and efficient, e.g., this is more robust and efficient compared with black-box analysis algorithms which need to learn for a given video to classify the correct surgery type in a single step. Such two-stage classification can also be easily extended to new types of microsurgical interventions, e.g., by simply modifying rule-based classification based on detected objects.

Summarizing, techniques have been disclosed that enable to filter the candidate types of a microsurgical intervention based on metadata of a microsurgical intervention. Then, in view of such pre-filtering, a domain-specific analysis of video data of the microsurgical intervention can be enabled. The appropriate model for performing this analysis can be selected.

Further summarizing, a method of surgery type classification for a video file from a predefined set of surgery types has been disclosed. The predefined set of surgery types may be related to a (single) defined anatomical part of a body, e. g. eye, brain, spine, jaw ("surgery specialty").

The surgery type classification uses a processing procedure for video files comprising at least the following steps of processing.

In a first step the video data is attributed to a subset of the predefined set of surgery types by analyzing metadata of the video file or generally associated with the surgery.

The metadata may include information about the surgery recording device; surgery recording device can be designed for video-capturing of a plurality of surgery types (e. g. surgical microscope).

The metadata can be external to an amount of pixel information in frames of the video data; e. g. in a video comprising multiple image frames the metadata can be associated with the video file, and not with the individual frames

The first step may additionally comprise an examination if statistical data on usage of a certain device in a certain clinic and/or at a certain location and/or by a certain surgeon is available for a video under examination and if this data is sufficient for calculating a probability of the surgery type of this video.

In a second step of the method, if the subset of the first step contains more than a single element, i.e., more than a single surgery type, the video file is analyzed using an (image/image series) analysis method specifically adapted to videos of the predefined set of surgery types, preferably adapted to the subset, i. e. the surgery types in the subset. A number of image property values of at least one predefined image property (e. g. grey value distribution, presence of specific tools, etc.) may be computed from a plurality of images (frames) of the video file.

More generally, it has been shown that image properties can include image properties, image characteristics and/or image features. Image characteristics are determined by statistical interpretation of information contained in a (pixel) image (i.e., on a computational level). Examples include a predefined range of statistical values, which can comprise brightness, contrast, color distribution, gray value histograms, count gradients/edges etc. On the other hand, image features are determined by detecting items displayed in an image, optionally which items, which size, where in the image, how often through the plurality of images (i.e., on a semantic level).

In a third step the video file is attributed to one single surgery type of the predefined set of surgery types (preferably of the subset). Such attribution method may be based on a pre-defined attribution scheme. The pre-defined attribution scheme may be dependent on surgery type probability of a given video relative to a pre-defined probability threshold. Alternatively or additionally, the pre-defined attribution scheme may be dependent on prior knowledge about the properties of individual surgery types, e.g., obtained from the metadata. For instance, statistical usage data can be additionally taken in account here if determined with a probability of less than 100 % or less than 99% or less than 95%, etc.

Summarizing, at least the following EXAMPLES have been disclosed.

EXAMPLE 1.A computer-implemented method, the method comprising:
- obtaining (3005) video data (151, 152, 153), the video data (151, 152, 153) encoding a microsurgical video comprising multiple frames that depict an intervention region of a microsurgical intervention,
- obtaining (3010) metadata (155, 156, 157) associated with the microsurgical intervention,
- establishing whether a type of the microsurgical intervention can be determined based on the metadata (155, 156, 157), and
- responsive to establishing that the type of the microsurgical intervention cannot be determined based on the metadata (155, 156, 157): performing an analysis of the video data (151, 152, 153) to determine the type (391) of the microsurgical intervention, the analysis of the video data (151, 152, 153) being based on the metadata (155, 156, 157).

EXAMPLE 2. The computer-implemented method of EXAMPLE 1,
wherein the analysis of the video data takes into account a non-probabilistic or probabilistic classification prior determined based on the metadata.

EXAMPLE 3. The computer-implemented method of EXAMPLE 1 or 2,
wherein the analysis of the video data (151, 152, 152) to determine the type (391) of the microsurgical intervention is configured based on the metadata (155, 156, 157).

EXAMPLE 4. The computer-implemented method of any one of the preceding EXAMPLEs, further comprising:
- performing an analysis of the metadata (155, 156, 157),
wherein the analysis of the video data (151, 152, 153) to determine the type (391) of the microsurgical intervention is based on an output of the analysis of the metadata (155, 156, 157) or is configured based on the output of the analysis of the metadata (155, 156, 157).

EXAMPLE 5. The computer-implemented method of EXAMPLE 3 or 4,
wherein the analysis of the video data (151, 152, 153) is configured by selecting an analysis algorithm for performing the analysis of the video data (151, 152, 153).

EXAMPLE 6. The computer-implemented method of EXAMPLE 5,
wherein the analysis algorithm for performing the analysis of the video data (151, 152, 153) is selected depending on one or more candidate types of the microsurgical intervention determined based on the metadata (155, 156, 157).

EXAMPLE 7. The computer-implemented method of any one of EXAMPLEs 3 to 6,
wherein the analysis of the video data (151, 152, 153) to determine the type of the microsurgical intervention is configured by determining a set of candidate types from which the analysis of the video data (151, 152, 153) selects the type.

EXAMPLE 8. The computer-implemented method of any one of the preceding EXAMPLEs,
wherein the metadata (155, 156, 157) is indicative of at least one of a medical imaging apparatus used to acquire the video data (151, 152, 153), a hardware and/or software setting of the medical imaging apparatus, usage information of the medical imaging apparatus, a surgical tool used in the microsurgical intervention, a user associated with the microsurgical intervention, a location where the microsurgical intervention was performed, or an institution where the microsurgical intervention was performed.

EXAMPLE 9. The computer-implemented method of any one of the preceding EXAMPLEs,
wherein the metadata (155, 156, 157) comprises probabilistic information.

EXAMPLE 10. The computer-implemented method of any one of the preceding EXAMPLEs, further comprising:
- responsive to establishing that the type of the microsurgical intervention can be determined based on the metadata (155, 156, 157): determining the type (391) of the microsurgical intervention based on the metadata (155, 156, 157) and without performing the analysis of the video data.

EXAMPLE 11. The computer-implemented method of EXAMPLE 10,
wherein said determining of the type (391) of the microsurgical intervention based on the metadata is based on a predefined mapping between values of one or more information elements of the metadata and types of the microsurgical intervention.

EXAMPLE 12. The computer-implemented method of any one of the preceding EXAMPLEs, further comprising:
- determining (3030) a representation of the video data (151, 152, 153),
wherein the analysis of the video data (151, 152, 153) to determine the type of the microsurgical intervention is performed based on the representation of the video data (151, 152, 153).

EXAMPLE 13. The computer-implemented method of EXAMPLE 12,
wherein the representation of the video data (151, 152, 153) aggregates information across at least some of the multiple frames of the microsurgical video.

EXAMPLE 14. The computer-implemented method of EXAMPLE 12 or 13,
wherein the representation of the video data (151, 152, 153) discards a temporal relationship of the multiple frames.

EXAMPLE 15. The computer-implemented method of any one of EXAMPLEs 12 to 14,
wherein the representation of the video data (151, 152, 153) comprises an identification of a semantic content of the microsurgical video.

EXAMPLE 16. The computer-implemented method of EXAMPLE 15,
wherein the identification of the semantic content of the microsurgical video is indicative of one or more of a presence of one or more objects of predefined types in the microsurgical video, a localization of one or more objects of predefined types in the microsurgical video, a spatial relationship between multiple objects in the microsurgical video, a count of one or more objects of predefined types in the microsurgical video, a duration of a visibility of one or more objects of predefined types in the microsurgical video, a sequence of appearance of one or more objects of predefined types in the microsurgical video.

EXAMPLE 17. The computer-implemented method of any one of EXAMPLEs 12 to 16,
wherein the representation of the video data (151, 152, 153) is based on a statistical characterization of characteristics of pixel values of at least some frames of the microsurgical video.

EXAMPLE 18. The computer-implemented method of EXAMPLE 17,
wherein the statistical characterization is an aggregate statistical characterization that aggregates across one or more characteristics of the pixel values of the at least some frames.

EXAMPLE 19. The computer-implemented method of EXAMPLE 17 or 18,
wherein the statistical characterization is limited in accordance with a predefined range of the one or more characteristics of the pixel values.

EXAMPLE 20. The computer-implemented method of EXAMPLE 18 or 19,
wherein the one or more characteristics of the pixel values comprises at least one of a distribution or a distribution section of pixel brightness values, pixel gray scale values, or pixel color values, or a contrast level.

EXAMPLE 21. The computer-implemented method of any one of EXAMPLEs 12 to 20, further comprising:
- identifying one or more characteristics of the representation of the video data (151, 152, 153), and
- selecting an analysis algorithm for performing the analysis of the representation based on said identifying of the one or more characteristics of the representation of the video data (151, 152, 153).

EXAMPLE 22. The computer-implemented method of any one of EXAMPLEs 12 to 21, further comprising:
- determining whether the representation of the video data (151, 152, 153) fulfills one or more predefined criteria.

EXAMPLE 23. The computer-implemented method of EXAMPLE 22, further comprising:
- responsive to determining that the representation of the video data (151, 152, 153) fulfills the one or more predefined criteria: selecting the type of the microsurgical intervention based on a predefined non-probabilistic ruleset analysis algorithm from candidate types, and
- responsive to determining that the representation does not fulfill the one or more predefined criteria: determining a prediction of the type of the microsurgical intervention based on a probabilistic analysis algorithm.

EXAMPLE 24. The computer-implemented method of EXAMPLE 22 or 23,
wherein the one or more predefined criteria comprise whether one or more objects of one or more types are visible and/or appear in a certain sequence in the microsurgical video.

EXAMPLE 25. The computer-implemented method of any one of EXAMPLEs 20 to 24,
wherein the one or more predefined criteria comprise whether a certain count or count range of one or more objects of one or more types are visible in the microsurgical video.

EXAMPLE 26. The computer-implemented method of any one of the preceding EXAMPLEs, further comprising:
- prior to performing the analysis of the video data (151, 152, 153), discarding (3025) at least some of the frames of the microsurgical video associated with at least one of a pre-intervention phase or a post-intervention phase of the microsurgical intervention.

EXAMPLE 27. The computer-implemented method of EXAMPLE 26,
wherein the at least some of the frames are discarded based on a cluster analysis of a statistical characterization of characteristics of pixel values of at least some frames of the video data (151, 152, 153).

EXAMPLE 28. The computer-implemented method of any one of the preceding EXAMPLEs,
wherein said performing of the analysis of the video data to determine the type (391) of the microsurgical intervention comprises applying a machine-learning classification algorithm,
wherein the machine-learning classification algorithm is optionally a random forest classifier.

EXAMPLE 29. The computer-implemented method of any one of the preceding EXAMPLEs, further comprising:
- determining a reliability of said determining of the type of the microsurgical intervention, and
- depending on the reliability, selectively obtaining (3205) user confirmation of the type of the microsurgical intervention.

EXAMPLE 30. The computer-implemented method of any one of the preceding EXAMPLEs,
wherein the type of the microsurgical intervention is determined across multiple specialties,
wherein the type of the microsurgical intervention is determined to discriminate between multiple types per specialty.

EXAMPLE 31. The computer-implemented method of any one of the preceding EXAMPLEs,
wherein an analysis algorithm used for said performing of the analysis of the video data to determine the type of the microsurgical intervention determines the type of the microsurgical intervention by selection from a predefined set of candidate types,
wherein the candidate types are associated with multiple hierarchy levels,
wherein the hierarchy levels comprise a first hierarchy level associated with different intervention regions,
wherein the hierarchy levels comprise a second hierarchy level associated with multiple intervention types for a given intervention region of the first hierarchy level.

EXAMPLE 32. The computer-implemented method of EXAMPLE 31,
wherein the predefined set of candidate types comprises: vitreo-retinal eye surgery and cataract eye surgery.

EXAMPLE 33. The computer-implemented method of any one of the preceding EXAMPLEs, further comprising:
- performing a further analysis of the microsurgical intervention using a type-specific analysis algorithm that is selected depending on the type of the microsurgical intervention.

EXAMPLE 34. The computer-implemented method of any one of the preceding EXAMPLEs, further comprising:
- transmitting a trigger signal that depends on the type of the microsurgical intervention.

EXAMPLE 35. The computer-implemented method of any one of the preceding EXAMPLEs,
wherein the video data (151, 152, 153) is obtained after completion of the microsurgical intervention,
wherein the microsurgical video covers a pre-intervention phase of the microsurgical intervention, an intervention phase of the of the microsurgical intervention, and a post-intervention phase of the of the microsurgical intervention.

EXAMPLE 36. The computer-implemented method of any one of the preceding EXAMPLEs,
wherein said establishing whether the type of the microsurgical intervention can be determined based on the metadata (155, 156, 157) depends on a level of granularity of discriminating between different types.

EXAMPLE 37. The computer-implemented method of EXAMPLE 36,
wherein the level of granularity is re-configurable.

EXAMPLE 38. The computer-implemented method of any one of the preceding EXAMPLEs,
wherein said establishing whether the type of the microsurgical intervention can be determined based on the metadata (155, 156, 157) is based on prior knowledge regarding a mapping between information included in the metadata (155, 156, 157) and types of the microsurgical intervention.

EXAMPLE 39. A computer-implemented method, comprising:
- obtaining video data encoding a microsurgical video of a microsurgical intervention,
- obtaining metadata associated with microsurgical intervention,
- implementing a multi-stage classification process to determine a type of the microsurgical intervention, the multi-stage classification process comprising a first stage and a second stage, the first stage being executed prior to the second stage,

wherein the first stage of the multi-stage classification process is based on the metadata and does not comprise an analysis of the microsurgical video,
wherein the second stage of the multi-stage classification process comprises the analysis of the microsurgical video.

EXAMPLE 40. The computer-implemented method of EXAMPLE 39,
wherein the second stage is selectively executed depending on an output of the first stage.

EXAMPLE 41. The computer-implemented method of EXAMPLE 40,
wherein the second stage is executed if the type of the microsurgical intervention cannot be unambiguously determined in the first stage.

EXAMPLE 42. The computer-implemented method of any one of EXAMPLEs 39 to 41,
wherein the second stage is based on the metadata.

EXAMPLE 43. The computer-implemented method of any one of EXAMPLEs 39 to 42,
wherein the second stage is configured based on the metadata.

EXAMPLE 44. The computer-implemented method of any one of EXAMPLEs 39 to 43,
wherein the second stage comprises multiple sub-stages, a first sub-stage of the second stage comprising determining a representation of the video data, a second sub-stage executed after the first sub-stage being based on the representation of the video data.

EXAMPLE 45. A processing device comprising a processor and a memory, the processor being configured to load program code from the memory and execute the program code, the processor, upon loading and executing the program code, executing the method of any one of the preceding EXAMPLEs.

EXAMPLE 46. Program code that is executable by a processor, the processor, upon loading and executing the program code, executing the method of any one of EXAMPLEs 1 to 44.

Although the invention has been shown and described with respect to certain preferred embodiments, equivalents and modifications will occur to others skilled in the art upon the reading and understanding of the specification. The present invention includes all such equivalents and modifications and is limited only by the scope of the appended claims.

For illustration, above various scenarios have been disclosed in which it is primarily attempted to determine the type of the microsurgical intervention based on metadata associated with microsurgical video. In other scenarios, such primary attempt to determine the type of the microsurgical intervention based on metadata is not performed; rather, the analysis algorithm is directly applied to the video data or a representation thereof. I.e., it is not required in all scenarios that a multi-stage processing pipeline is used for determining the type of the microsurgical intervention.

For further illustration, above various examples have been disclosed in which the type of the microsurgical intervention is determined based on video data of a microsurgical video and/or metadata associated therewith. As a general rule, instead of using video data that depicts an intervention region of the microsurgical intervention, it would also be possible that the video data depicts an operating room of the microsurgical intervention. Furthermore, further inputs can be provided to an analysis algorithm that determines the type of the microsurgical intervention, e.g., context data associated with a context of the microsurgical intervention. For instance, one or more sensor values or state data of operating equipment can be additionally provided to reliably determine the type of the microsurgical intervention.

## Claims

1. A computer-implemented method, the method comprising:
- obtaining (3005) video data (151, 152, 153), the video data (151, 152, 153) encoding a microsurgical video comprising multiple frames that depict an intervention region of a microsurgical intervention,
- obtaining (3010) metadata (155, 156, 157) associated with the microsurgical intervention,
- establishing whether a type of the microsurgical intervention can be determined based on the metadata (155, 156, 157), and
- responsive to establishing that the type of the microsurgical intervention cannot be determined based on the metadata (155, 156, 157): performing an analysis of the video data (151, 152, 153) to determine the type (391) of the microsurgical intervention, the analysis of the video data (151, 152, 153) being based on the metadata (155, 156, 157).

2. The computer-implemented method of claim 1,
wherein the analysis of the video data takes into account a non-probabilistic or probabilistic classification prior determined based on the metadata.

3. The computer-implemented method of claim 1 or 2,
wherein the analysis of the video data (151, 152, 152) to determine the type (391) of the microsurgical intervention is configured based on the metadata (155, 156, 157).

4. The computer-implemented method of claim 3,
wherein the analysis of the video data (151, 152, 153) is configured by selecting an analysis algorithm for performing the analysis of the video data (151, 152, 153).

5. The computer-implemented method of claim 4,
wherein the analysis algorithm for performing the analysis of the video data (151, 152, 153) is selected depending on one or more candidate types of the microsurgical intervention determined based on the metadata (155, 156, 157).

6. The computer-implemented method of any one of claims 3 to 5,
wherein the analysis of the video data (151, 152, 153) to determine the type of the microsurgical intervention is configured by determining a set of candidate types from which the analysis of the video data (151, 152, 153) selects the type.

7. The computer-implemented method of any one of the preceding claims,
wherein the metadata (155, 156, 157) is indicative of at least one of a medical imaging apparatus used to acquire the video data (151, 152, 153), a hardware and/or software setting of the medical imaging apparatus, usage information of the medical imaging apparatus, a surgical tool used in the microsurgical intervention, a user associated with the microsurgical intervention, a location where the microsurgical intervention was performed, or an institution where the microsurgical intervention was performed.

8. The computer-implemented method of any one of the preceding claims, further comprising:
- determining (3030) a representation of the video data (151, 152, 153),
wherein the analysis of the video data (151, 152, 153) to determine the type of the microsurgical intervention is performed based on the representation of the video data (151, 152, 153).

9. The computer-implemented method of claim 8,
wherein the representation of the video data (151, 152, 153) aggregates information across at least some of the multiple frames of the microsurgical video.

10. The computer-implemented method of claim 8 or 9,
wherein the representation of the video data (151, 152, 153) comprises an identification of a semantic content of the microsurgical video.

11. The computer-implemented method of claim 10,
wherein the identification of the semantic content of the microsurgical video is indicative of one or more of a presence of one or more objects of predefined types in the microsurgical video, a localization of one or more objects of predefined types in the microsurgical video, a spatial relationship between multiple objects in the microsurgical video, a count of one or more objects of predefined types in the microsurgical video, a duration of a visibility of one or more objects of predefined types in the microsurgical video, a sequence of appearance of one or more objects of predefined types in the microsurgical video.

12. The computer-implemented method of any one of claims 8 to 11, further comprising:
- determining whether the representation of the video data (151, 152, 153) fulfills one or more predefined criteria.

13. The computer-implemented method of claim 12,
wherein the one or more predefined criteria comprise whether one or more objects of one or more types are visible and/or appear in a certain sequence in the microsurgical video.

14. The computer-implemented method of any one of the preceding claims,
wherein the type of the microsurgical intervention is determined across multiple specialties,
wherein the type of the microsurgical intervention is determined to discriminate between multiple types per specialty.

15. The computer-implemented method of any one of the preceding claims, further comprising:
- performing a further analysis of the microsurgical intervention using a type-specific analysis algorithm that is selected depending on the type of the microsurgical intervention.

16. A processing device comprising a processor and a memory, the processor being configured to load program code from the memory and execute the program code, the processor, upon loading and executing the program code, executing the method of any one of the preceding claims.
